# EUROPÄISCHE PATENTSCHRIFT

(11) **EP 3 622 052 B1**
(45) Veröffentlichungstag und Bekanntmachung des Hinweises auf die Patenterteilung: **05.04.2023**
(21) Anmeldenummer: 18715504.9
(22) Anmeldetag: 15.03.2018
(51) Int. Cl.: C12N 5/00

(54) **NEURONAL INDUKTIVE KULTIVIERUNGSMATRIX**
NEURONALLY INDUCTIVE CULTIVATION MATRIX
MATRICE DE CULTURE PAR INDUCTION NEURALE

(30) Priorität: 08.05.2017 DE 102017207698
(43) Veröffentlichungstag der Anmeldung: 18.03.2020
(73) Patentinhaber: Fraunhofer-Gesellschaft zur Förderung der angewandten Forschung e.V., 80686 München (DE)
(72) Erfinder: GUTERMUTH, Angela, 52074 Aachen (DE); BAUM, Christoph, 50859 Köln (DE)
(74) Vertreter: Schrell, Andreas
(86) Internationale Anmeldenummer: PCT/EP2018/056590
(87) Internationale Veröffentlichungsnummer: WO 2018/206185

(56) Entgegenhaltungen:
- DE-A1-102015 205 534
- JP-A- 2017 055 761
- Claudia Skazik-Voogt ET AL: "Fertigungsverfahren für das Tissue Engineering", GIT-Labor - Portal für Anwender in Wissenschaft und Industrie, 12. April 2017 (2017-04-12), XP055471890, Gefunden im Internet: URL:https://www.git-labor.de/forschung/lif e-sciences-biotechnologie/fertigungsverfah ren-fuer-das-tissue-engineering [gefunden am 2018-05-02]
- Angela Gutermuth: "Masterarbeit Life Sciences Engineering", , 1. November 2015 (2015-11-01), XP055471842, Gefunden im Internet: URL:https://fsbio.rwth-aachen.de/sites/def ault/files/fsfiles/Ausschreibung%20Mastera rbeit_Fraunhofer%20IPT.pdf [gefunden am 2018-05-02]

## Beschreibung

Die Erfindung betrifft Verfahren zur Herstellung einer Matrix zur Differenzierung in neuronale Zellen und neuronales Gewebe aus isolierten Zellen, besonders aus Stammzellen, außerhalb des menschlichen oder tierischen Körpers.

In der Forschung und Therapie besteht der Bedarf nach neuronalen Zellen und neuronalem Gewebe aus humanem und autologem Zellmaterial zum Zwecke der Zelltherapie und der patientenspezifischen Medikamentenentwicklung. Es besteht auch der Wunsch nach genotypisch identifizierbaren und festlegbaren neuronalen Zelllinien für Forschungszwecke.

Verfahren zur Herstellung von neuronalen Zellen oder Geweben aus Stammzellen oder anderen pluripotenten oder plastischen Zellen sind bekannt. Diese beruhen auf xenobiotischen Differenzierungsfaktoren. Der Einsatz solcher Faktoren führt aber zu nachteiligen Veränderungen an den zu differenzierenden Zellen und erschwert auch deren Zulassung, beispielsweise als Medizinprodukt oder Therapeutikum. Andere bekannte Verfahren zur Herstellung autologer neuronaler Zellen basieren auf der Reprogrammierung adulter Gewebezellen in einen pluripotenten Status, welcher dann in die angestrebte neuronale Zelle differenziert wird. So können aus somatischen Fibroblasten zum Beispiel so genannte IPS-Zellen reprogrammiert werden. Bekannte Reprogrammierungsverfahren basieren aber auf Virustransformation. Daher sind die so hergestellten pluripotenten Zellen nicht ohne weiteres ohne Nebenwirkungen einsetzbar und werden nicht als Medizinprodukt oder Therapeutikum zugelassen. Außerdem ist die Reprogrammierungseffizienz von adulten Fibroblasten in neuronale Zellen sehr gering und liegt nach Untersuchungen bei unter 0,2%. Die Differenzierung dieser pluripotenten Zellen in neuronale Zellen wird mit Hilfe von chemischen neuronalen Differenzierungsfaktoren und/oder durch virale Transfektion erzielt. Die durch virale Transfektion generierten neuronalen Zellen können nur für die Medikamententestung eingesetzt werden, da sie krebserregend sein können.

Ein Verfahren, welches auf chemische Differenzierungsfaktoren oder gar virale Faktoren weitgehend oder vollständig verzichten kann, ist die Mechanotransduktion. Dabei werden Stammzellen oder andere pluripotente oder plastische Zellen durch spezifische mechanische Stimulation zur Ausbildung einer zelltypischen Topographie und letztlich zelltypischen Funktion stimuliert. Es bestehen erste Ansätze zur mechanotransduktiven neuronalen Differenzierung von Stammzellen. Diese basieren auf photolithographisch erzeugten Strukturen, die zum Beispiel auf Silikonelastomer (Polydimethylsiloxan, PDMS) abgeformt sind. Solche Verfahren erlauben zum einen nur die Herstellung von sehr kleinen Flächen, sodass nur geringe Mengen an neuronalen Zellen ausdifferenziert werden können. Außerdem hat sich dort die Effizienz der Differenzierung als nicht ausreichend erwiesen, und die Qualität von Funktion und Struktur der tatsächlich differenzierten Zellen als Nervenzellen ist verbesserungswürdig.

Der vorliegenden Erfindung lag das technische Problem zugrunde, Verfahren und Mittel zur Herstellung von neuronalen Zellen oder Geweben aus isolierten biologischen Zellen, insbesondere Stammzellen bereitzustellen, die eine unkomplizierte und wirtschaftlich attraktive Herstellung von insbesondere autologen neuronalen Zellen oder Geweben in großen Mengen und hoher Qualität ermöglichen. Dazu war es erforderlich, sowohl bekannte Verfahren der mechanotransduktiven Differenzierung in Bezug auf die Effizienz und Qualität der Differenzierung zu neuronalen Zellen zu verbessern, als auch Verfahren zu entwickeln, welche die Herstellung einer großen Zahl von neuronalen Zellen oder Geweben ermöglichen.

Die Erfindung löst das zugrundeliegende technische Problem durch die Bereitstellung eines Verfahrens zur Herstellung einer Kultivierungsmatrix (Kultivierungssubstrat) zur Kultivierung von darauf ausgesäten biologischen, insbesondere pluripotenten oder plastischen, Zellen und zur Differenzierung dieser Zellen in neuronale Zellen oder neuronale Gewebe. Dabei besteht die hergestellte Matrix zumindest oder bevorzugt ausschließlich aus einem Grundkörper aus biokompatiblem Polymer oder Polymer biologischer Herkunft (biologischem Polymer), welches eine erfindungsgemäß strukturierte Oberfläche aufweist. Diese besondere Oberflächenstruktur ist gekennzeichnet durch eine periodische Mikrostruktur aus parallelen mikrodimensionierten Nuten oder Rillen, oder alternativ Stege, und einer periodischen Nanostruktur aus parallelen nanodimensionierten Nuten oder Rillen, oder alternativ Stege, wobei erfindungsgemäß die periodische Nanostruktur zu der periodischen Mikrostruktur parallel verläuft und die periodische Nanostruktur in die Mikrostruktur regelmäßig, das heißt in die Periode der Mikrostruktur, das heißt in die Nuten oder Rillen, oder alternativ auf den Stegen, der Mikrostruktur eingebettet ist. Das heißt besonders, periodische Mikrostruktur und periodische Nanostruktur wechseln sich auf der strukturierten Oberfläche ab. Ein regelmäßiges Muster aus parallel zueinander orientierten Nuten, Rillen oder Stegen ist dabei erfindungsgemäß bevorzugt.

Es zeigt sich überraschend, dass sich die auf dieser spezifischen strukturierten Oberfläche ausgesäten biologischen Zellen im Laufe der Kultivierung in ihrem Wachstum entlang der parallelen sowohl mikro- als auch nanodimensionierten Nuten oder Stegen derart orientieren, dass sich durch die so ausgelöste Mechanotransduktion aus den ausgesäten einzelnen Zellen neuronale Zellen und letztlich auch neuronale Gewebe mit eindeutig neuronaler Struktur und neuronaler Funktion stabil und zuverlässig entwickeln. Die Differenzierungseffizienz an der erfindungsgemäß strukturierten Oberfläche der hergestellten Kultivierungsmatrix ist außerordentlich hoch.

Diese positiven Effekte lassen sich noch steigern. In einer bevorzugten Ausgestaltung weist das biokompatible oder biologische Polymer des Grundkörpers zumindest im Bereich der strukturierten Oberfläche eine Steifigkeit (mechanische Festigkeit) auf, die der entsprechenden mechanischen Eigenschaft (Steifigkeit) der biologischen Zellen entspricht und bevorzugt nicht größer, besonders bevorzugt kleiner als diese ist. Es hat sich in Abkehr von bisheriger Vorstellung gezeigt, dass durch eine strukturierte Oberfläche, die vergleichsweise nachgiebig und weniger steif ist, die Effizienz der Mechanotransduktion und die Qualität der erzeugten neuronalen Zellen und Gewebe deutlich verbessern lässt. Bevorzugt ist die Steifigkeit der strukturierten Oberfläche der Matrix durch die Wahl des biokompatiblen oder biologischen Polymers und/oder dessen Zusammensetzung oder Weise der Herstellung bestimmt. Bekanntermaßen lassen sich solche Polymere in ihren mechanischen Eigenschaften durch Wahl der Zusammensetzung und/oder die Art und Weise der Vernetzung einstellen. Geeignete Polymermaterialien sind nachfolgend beschrieben. Bevorzugt ist eine gelartige Polymermatrix.

Die Erfinder fanden überraschend, dass durch eine ganz spezifische Dimensionierung der parallelen Nuten oder Rillen, oder alternativ Stege, der erfindungsgemäß mikro- und nanostrukturierten Oberfläche der hergestellten Matrix eine besonders hohe Differenzierungseffizienz und Qualität der erhaltenen differenzierten neuronalen Zellen und Gewebe erreicht werden kann. Überraschend wurde dabei gefunden, dass das insbesondere streng periodische Abwechseln einer niedrig periodischen Mikrostruktur mit einer höher periodischen Nanostruktur den besonderen Differenzierungserfolg ermöglicht. Das heißt, mehrere Perioden der Nanostruktur sind in einer Periode der Mikrostruktur eingebettet. Bevorzugt ist die Mikrostruktur aus Nuten oder Rillen, oder alternativ aus Stegen, mit einer Tiefe, beziehungsweise Höhe, von 0,7 bis 1,5 µm und einer Breite von 1 bis 2 µm gebildet. Die einzelnen Nuten oder Rillen, oder alternativ Stege, der Mikrostruktur besitzen einen Mittenabstand (Periode) von 3 bis 12 µm zueinander. Es hat sich gezeigt, dass hier die Dimensionierung der Strukturen von hoher Bedeutung ist. In besonderer Ausführung sind die Nuten der Mikrostruktur als Rillen ausgebildet. In alternativer Ausgestaltung sind anstatt der Nuten der Mikrostruktur entsprechend dimensionierte Stege ausgebildet (Negativform).

Bevorzugt ist die Nanostruktur, die in der vorgenannten Mikrostruktur eingebettet ist, aus Nuten oder Rillen, oder alternativ Stege, mit einer Tiefe, beziehungsweise Höhe, von 15 bis 35 µm und einer Breite 450 bis 550 µm gebildet. Die einzelnen Nuten oder Rillen, oder alternativ Stege, der Nanostruktur besitzen einen Mittenabstand (Periode, d.h. Pitch) von 500 bis 550 µm zueinander. Es hat sich gezeigt, dass hier die Dimensionierung der Strukturen von hoher Bedeutung ist. In besonderer Ausgestaltung sind die Nuten der Nanostruktur als Rillen ausgebildet. In alternativer Ausgestaltung sind anstatt der Nuten der Nanostruktur entsprechend dimensionierte Stege ausgebildet (Negativform).

In einer besonderen Variante ist die Mikrostruktur als Nuten oder Rillen ausgebildet und die in die Nut oder Rille der Mikrostruktur eingebettete Nanostruktur als Nuten oder Rillen ausgebildet. In einer alternativen Variante ist die Mikrostruktur als Nuten oder Rillen ausgebildet und die in die Nut oder Rille der Mikrostruktur eingebettete Nanostruktur als Stege ausgebildet. In einer alternativen Variante ist die Mikrostruktur als Stege ausgebildet und die auf dem Steg der Mikrostruktur eingebettete Nanostruktur als Nuten oder Rillen ausgebildet. In einer alternativen Variante ist die Mikrostruktur als Stege ausgebildet und die auf dem Steg der Mikrostruktur eingebettete Nanostruktur als Stege ausgebildet.

Der Gegenstand der Erfindung ist ein neuartiges Verfahren zur Herstellung einer solchen neuronal induktiven Kultivierungsmatrix, wobei die strukturierte Oberfläche der Matrix durch mikrostrukturierende spanabhebende Verfahren erzeugt wird. Erfindungsgemäß bevorzugt ist dabei, dass die periodische Mikrostruktur und die darin eingebettete periodische Nanostruktur gebildet werden, und zwar zusammen in einem einzigen Arbeitsschritt des Mikrostrukturierens.

Das erfindungsgemäße Mikrostrukturierverfahren ist ein spanabhebendes Verfahren mit einem relativ zum bearbeiteten Werkstoff bewegten Schneidwerkzeug, und zwar bevorzugt ausgewählt aus: Hobeln, Stoßen und Fräsen. Bevorzugt ist das spanabhebende Verfahren ein Verfahren mit einem feststehenden Schneidwerkzeug mit sich bewegendem zu bearbeitetem Werkstoff, und zwar besonders Drehen. Besonders bevorzugt ist das Mikrostrukturierverfahren Drehen mit einem Drehmeißel

Erfindungsgemäß wird diese spanabhebende Bearbeitung mit einem Schneidwerkzeug, das heißt spezifisch Klinge, Drehmeißel oder Fräser, durchgeführt, das eine Schneide mit einen Spitzenradius (r_{E}) von weniger als 10 µm besitzt und aus monokristallinem Diamant aufgebaut ist. Es hat sich überraschend gezeigt, dass durch Verwendung eines solchen Schneidwerkzeugs mit einer derartigen Schneide in besonders einfacher und zuverlässiger Weise die hierin beschriebene spezifische Strukturierung erhalten werden kann. Die Nanostruktur kann dabei bevorzugt in einem einzigen Bearbeitungsschritt zusammen mit der Mikrostruktur erzeugt werden. Die Nanostruktur ist dabei unmittelbar von dem Vorschub des Schneidwerkzeugs (pro Umdrehung bzw. pro Hub) sowie dem Spitzenradius (r_{E}) abhängig.

Bevorzugt beträgt der Spitzenradius (r_{E}) der Werkzeugschneide 5 µm oder weniger als 5 µm, 2 µm oder bevorzugt weniger als 2 µm oder 1 µm oder bevorzugt weniger als 1 µm, oder 500 nm oder weniger als 500 nm. In einer Variante ist das Schneidwerkzeug spitz, in einer anderen Variante ist das Schneidwerkzeug facettiert. Eine Schleppschneide ist bevorzugt vermieden. Bevorzugt ist ein MDC-Schneidwerkzeug mit einem 1x30 ° Spitzenwinkel und Werkzeugradius (r_{E}) von 2 µm oder 1 µm. Ein Vorschub von 500 bis 550 nm, entsprechend der Periode (Pitch) der zu erzeugenden Nanostrukturierung.

Es wird offenbart, dass diese spanabhebende Mikrostrukturierung mittels dieses Verfahrens direkt an dem polymeren Grundkörper der Matrix angewandt werden kann, um die strukturierte neuronal induktive Oberfläche direkt zu erhalten. Erfindungsgemäß wird diese Oberflächenstruktur als Negativform zunächst an einem Werkzeug ausgebildet und dann anschließend die eigentliche neuronal induktive Oberfläche an dem Grundkörper der Matrix aus biokompatiblem oder biologischem Polymer mittels dieses oberflächenstrukturierten Werkzeugs erzeugt. Erfindungsgemäß wird das Polymer des Grundkörpers, bevorzugt in noch flüssiger Form, auf das oberflächenstrukturierte Werkzeug, das heißt zumindest auf die an dem Werkzeug nach der Mikrostrukturierung ausgebildete strukturierte Oberfläche, aufgegossen. Erfindungsgemäß wird das Polymer des Grundkörpers mit diesem Werkzeug geprägt, sodass sich die strukturierte Oberfläche an dem Grundkörper ausbildet. Dabei ist gegebenenfalls jeweils vorgesehen, dass das Polymer an der strukturierten Oberfläche dieses Werkzeugs selbst aushärtet oder trocknet und anschließend die so oberflächenstrukturierte Matrix in ausgehärteter und/oder getrockneter Form von dem Werkzeug getrennt, das heißt, insbesondere davon abgehoben wird. In bevorzugter Ausgestaltung wird in einem weiteren Schritt zur Verwendung der Matrix der ausgehärtete oder insbesondere der getrocknete polymere Grundkörper mit Flüssigkeit, besonders Kulturmedium, in Kontakt gebracht wobei dadurch seine gewünschte Endform und mechanische Steifigkeit durch Aufquellen und Hydratisieren erzeugt wird. Bevorzugt ist vorgesehen, den Grundkörper in trockener Form zu konfektionieren und zu lagern und die Quellung und Hydratisierung erst unmittelbar bei Benutzung zur Kultivierung von Zellen vorzunehmen. Das oberflächenstrukturierte Werkzeug kann wiederkehrend zur Herstellung weiterer neuronal induktiver Kultivierungsmatrices der Erfindung eingesetzt werden. Die Kultivierungsmatrices können mittels dieses Präge- oder Abgussverfahrens in großer Zahl leicht hergestellt werden. Außerdem erlaubt das einfache Strukturierungsverfahren die Bereitstellung flächenmäßig großer neuronal induktiver Kultivierungsmatrices, so dass auch großflächige neuronale Zellverbände und neuronale Gewebe hergestellt werden können.

In einer besonderen Ausgestaltung ist das oberflächenstrukturierte Werkzeug also als Prägestempel oder aber als Prägewalze ausgebildet die Aufprägung der erfindungsgemäß strukturierten Oberfläche auf flüssiges oder gerade aushärtendes Polymergel erfolgt ähnlich wie in der Drucktechnik. Auch so lassen sich große Mengen und große Flächen an Kultivierungsmatrix zur Herstellung von neuronalen Zellen oder Geweben herstellen.

Das biokompatible Polymer sollte hydrophil genug sein, um Zelladhäsion zu ermöglichen. Bevorzugt ist das biokompatible oder biologische Polymer ausgewählt der Gruppe der Biomaterialien, bestehend aus Kollagen, Agarose sowie aus Derivaten davon und halbsynthetischen Modifikationen davon, wie meta-acrylierte Gelatine (GelMA). Bevorzugt sind Polymere, welche zunächst flüssig vorliegen, bevor sie einen gelartigen Zustand einnehmen. Besonders bevorzugt ist lyophilisiertes Kollagen. Alternativ bevorzugt ist meta-acrylierte Gelatine (GelMA).

Besonders bevorzugt ist, ein Verfahren zur Herstellung von Matrices mit neurotransduktiver Topographie, wobei ein flüssiges Polymer, besonders eine Lösung lyophilisiertem Kollagen, auf ein Werkzeug mit erfindungsgemäß strukturierter Oberfläche aufgegossen wird, dort ausgehärtet wird und getrocknet wird. Die Trocknungsdauer ist abhängig vom Volumen, der Oberfläche und der Art des Gels. Die Trocknung erfolgt bevorzugt mittels Exsikkator. Die abgeformte erfindungsgemäße neuroinduktive Topographie bleibt bei den einsetzbaren Matrixsubstraten ohne weiteres über einige Wochen erhalten. Auf diese Weise lassen sich einfach und in großer Zahl Matrices zur Ausbildung von neuronalen Zellen oder Geweben herstellen und für die Benutzung bevorraten. Bei Aussaat der Zellen in Suspension quillt die getrocknete Gelmatrix und zeigt dann ihre ursprünglich eingeprägte neuroinduktive Oberflächenstrukturierung.

Als Abguss- oder Prägewerkzeug eignen sich insbesondere elastische Materialien wie Silikonelastomer (RTV) oder Polyurethane. Alternativ können wenig elastische oder unelastische flexible Folien oder starre Materialien verwendet werden. Bevorzugt sind als Werkstoff des Abguss- oder Prägewerkzeugs Metalle vorgesehen. Diese sind bevorzug ausgewählt aus Nichteisenmetallen, besonders Kupfer, kupferhaltige oder nickelhaltige Legierungen. Alternativ bevorzugt sind als Werkstoff des Abguss- oder Prägewerkzeugs Kunststoffe vorgesehen.

Besonders bevorzugt ist ein Prägewerkzeug das ein Metall- oder Kunststoffzylinder ist, der mittels Drehen mit dem hierin beschriebenen Schneidwerkzeug gedreht und damit mit Mikro- und Nanostruktur versehen wird. In dieser bevorzugten Ausführung ist das Abformverfahren zur Herstellung der oberflächenstrukturieren Matrix ein Folienprägeverfahren.

Die hierin beschriebene neuronal induktive Matrix kann zur mechanotransduktiven Differenzierung von Stammzellen zu neuronalem Gewebe verwendet werden.

Schließlich offenbart ist ein neuartiges Verfahren zur Herstellung von neuronalen Zellen oder Geweben aus isolierten biologischen Zellen, spezifisch aus Stammzellen, pluripotenten oder plastischen bereit. Dieses Verfahren enthält zumindest die Schritte des Inkontaktbringens isolierter biologischer Zellen mit der neuronal induktiven Matrix, hergestellt gemäß der vorliegenden Erfindung, anschließendes Kultivieren der biologischen Zellen auf oder an der strukturierten Oberfläche dieser Matrix, wobei eine mechanotransduktive Differenzierung dieser Zellen zu neuronalen Zellen und/oder neuronalem Gewebe erfolgt, wobei differenzierte neuronale Zellen und/oder neuronales Gewebe erhalten werden.

Bevorzugt ist vorgesehen, die neuronalen Zellen oder Gewebe in dieser Matrix eingebettet weiterzuverwenden. In einer alternativen Ausgestaltung werden die neuronalen Zellen oder das neuronale Gewebe von der Matrix in an sich bekannter Weise abgelöst. Gegebenenfalls werden die neuronalen Zellen von der Matrix abgelöst und dann zu einer Suspension vereinzelt. Die Suspension neuronaler Zellen kann weiter kultiviert werden.

Die erfindungsgemäß hergestellte Kultivierungsmatrix erlaubt vorteilhafterweise auch die Differenzierung von weniger plastischen Zellen auch aus adultem Gewebe. Bevorzugt sind Zellen aus dem Fettgewebe, aus dem Knochenmark und aus der Haut. Bevorzugt sind auch aus Nabelschnurblut gewonnene Zellen. Bevorzugt sind die isolierten biologischen Zellen, die zu neuronalen Zellen differenzieren sollen, Stammzellen, bevorzugt mesenchymale Stammzellen (MSC); es werden so differenzierte, insbesondere autologe, MSC erhalten. Mesenchymale Stammzellen können beispielsweise aus dem Fettgewebe eines autologen Spenders entnommen und isoliert werden. Der Einsatz solcher Zellen zur Therapie ist zugelassen und ethisch voll vertretbar. Ein Patient wird unmittelbar mit seinen eigenen Zellen aus seinem Fettgewebe behandelt.

Diese gewonnenen neuronalen Zellen oder Gewebe lassen sich als insbesondere patientenspezifische Zellen, das heißt als Medizinprodukt, unmittelbar in Therapie und Prophylaxe des Patienten einsetzen.

Die gewonnenen neuronalen Zellen oder Gewebe lassen sich auch als insbesondere patientenspezifische Zellen, auch zur Medikamentenentwicklung, besonders auch zur Entwicklung patientenspezifischer Medikamente, zur Therapie und Prophylaxe des Patienten einsetzen.

Die Erfindung wird nachfolgend näher beschrieben:

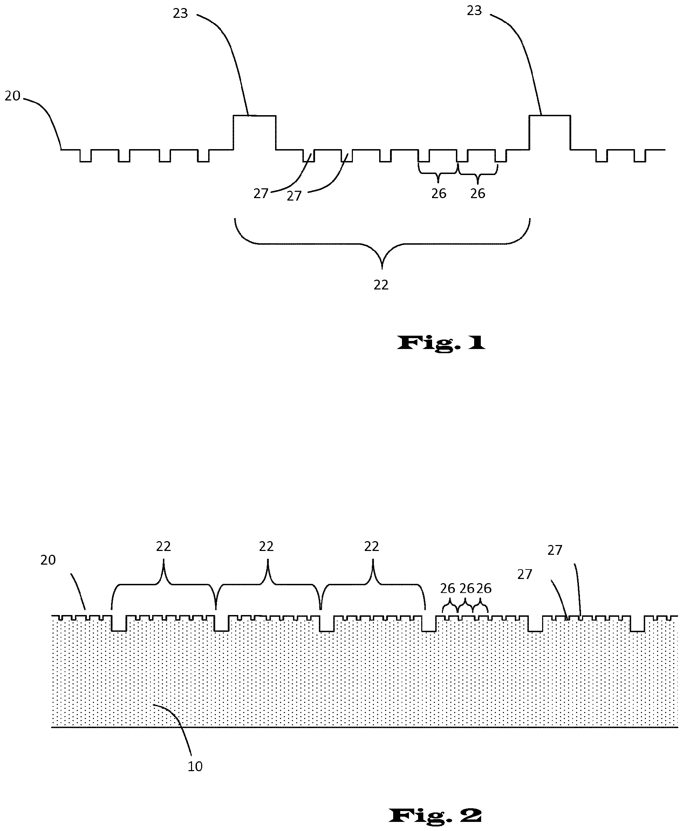

Figur 1 zeigt einen schematischen Querschnitt durch eine hergestellte neurotransduktive Kultivierungsmatrix der Erfindung in Detailansicht. Diese besteht aus einem Grundkörper mit einer strukturierten Oberfläche (20). Die Oberfläche (20) ist charakterisiert durch eine Mikrostruktur (22) aus parallelen mikrodimensionierten Stegen (23) und einer dazwischen eingebetteten periodischen Nanostruktur (26) mit parallelen nanodimensionierten Nuten (27) die zu der Mikrostruktur (22) parallel verläuft.
Figur 2 zeigt einen schematischen Querschnitt durch eine andere Variante einer hergestellten neurotransduktiven Kultivierungsmatrix der Erfindung in anderer Vergrößerung. An dem Grundkörper (10) der Matrix ist auf einer Seite eine strukturierte Oberfläche (20) ausgebildet. Diese ist charakterisiert durch eine periodische Mikrostruktur (22) mit parallelen mikrodimensionierten Nuten (23) und einer periodischen Nanostruktur (26) mit parallelen nanodimensionierten Nuten (27) die zu der Mikrostruktur (22) parallel verläuft, wobei die periodische Nanostruktur (26) in die Nuten (23) der Mikrostruktur (22) eingebettet ist.

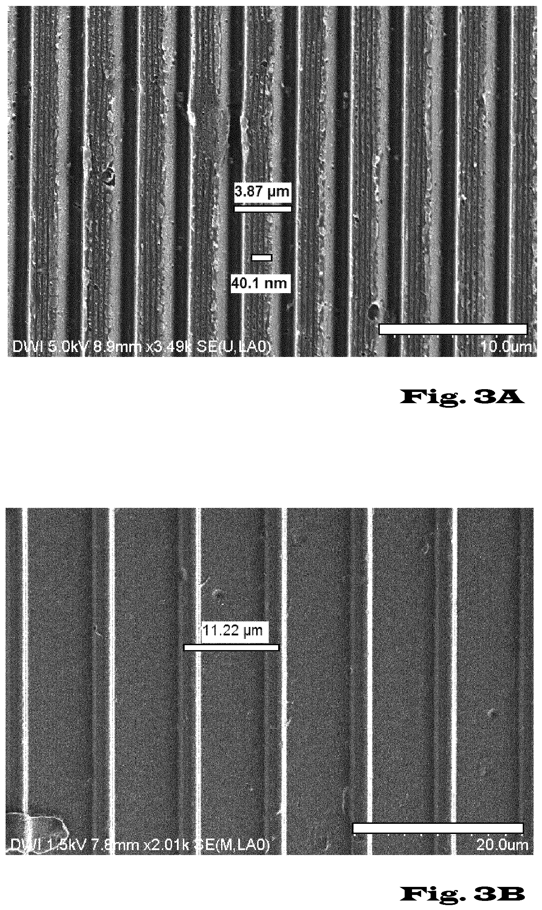

Figuren 3A und 3B zeigen elektronenmikroskopische Aufnahmen von erfindungsgemäßen oberflächenstrukturierten Kultivierungsmatrices in Draufsicht (Maßstäbe eingezeichnet). Figur 3A zeigt eine Kultivierungsmatrix mit der Struktur gemäß der schematischen, nicht maßstabsgerechten Darstellung nach Figur 1.

### Beispiel

Die gesamte Prozessfolge für die mechanotransduktive Differenzierung von Stammzellen in neuronale Zellen oder neuronale Gewebe beinhaltet (a) die Fertigung eines Masterwerkzeugs mit dem negativ der erfindungsgemäßen Topographie, (b) die Übertragung der Topographie auf ein abformbares Material, (c) gegebenenfalls weitere Abformungen von dem Masterwerkzeug und (d) Kultivierung von Stammzellen auf dem abgeformten strukturierten Material.

Zur Herstellung eines oberflächenstrukturierten Masterwerkzeugs wird eine Zylinderwalze aus Kupfer oder Nickel (oder entsprechenden Legierungen) in eine Drehmaschine gespannt und mittels eines Drehmeißels mit einer MDC-Schneide mit 1× 30° Spitzenwinkel und einem Spitzenradius von 1 µm bei einer Drehzahl von 150 min⁻¹ abgedreht. Das Schneidwerkzeug wird dabei so geführt, dass an dem Zylinder Stege mit einer Breite von 1,2 µm und einem Abstand von 11,2 µm geschnitten werden (Topographie 1). Alternativ wird der Vorschub so eingestellt, dass an dem Zylinder Stege mit einer Breite von 1,24 µm und einem Abstand von 3,8 µm erzeugt werden (Topographie 2). Die Stege bilden die Mikrostruktur.

Zum Schneiden wird ein Vorschub von 0,5 mm pro Umdrehung (bzw. Werkzeughub) gewählt. An dem Metallzylinder bildet sich zwischen den Stegen der Mikrostruktur eine Nanostruktur mit Rillen von 32 nm, die zueinander 500 nm beabstandet sind (Topographie 1) beziehungsweise Rillen mit einer Tiefe von 19,15 nm, die 40 nm zueinander beabstandet sind (Topographie 2).

Das so hergestellte oberflächenstrukturierte Masterwerkzeug wird als Prägewalze in eine Apparatur zur Prägung von Kollagenfolien eingespannt. Als Kollagenfolie wird ein Grundkörper aus frisch gegossenem lyophilisiertem Kollagen eingesetzt. Die Rillenstruktur der Walze wird in die Oberfläche der Kollagenfolie eingeprägt. Anschließend wird die Kollagenfolie mit strukturierter Oberfläche in einem Exsikkator durch Wasserentzug getrocknet.

In einem alternativen Ansatz wird durch Hobeln mit einem entsprechenden Schneidwerkzeug eine entsprechend strukturierte flache Metallplatte hergestellt. Diese dient als Schablone. Auf ihr wird eine frisch hergestellte flüssige Lösung aus lyophilisiertem Kollagen aufgegossen und dort aushärten lassen. Anschließend wird die hergestellte Kollagenfolie im Exsikkator für etwa vier Tage getrocknet. Dabei schrumpft die Folie um einige Prozent. Durch Schrumpfung kann sie von der Schablone leicht abgelöst werden.

In einem weiteren alternativen Ansatz wird durch Folienprägen oder Abgießen mittels Metallmasterwerkzeug eine entsprechend strukturierte flache Abgussform aus Silikonelastomer hergestellt. Diese Abgussform wird dann entsprechend als Schablone eingesetzt.

Auf ihr wird die flüssige Lösung aus lyophilisiertem Kollagen aufgegossen und dort aushärten lassen. Anschließend wird die hergestellte Kollagenfolie im Exsikkator für etwa vier Tage getrocknet. Dabei schrumpft die Folie um einige Prozent. Durch Schrumpfung kann sie von der Schablone leicht abgelöst werden. Alternativ wird die Abgussform aufgrund ihrer Elastizität von dem erstarrten oder getrockneten Kollagengel abgezogen.

Es hat sich gezeigt, dass sich die geprägte oder gegossene Kollagenfolie nach Trocknung für mehrere Wochen lagern lässt. Vorliegend wurde nach einer Lagerungszeit von vier Wochen die geprägte Folie angefeuchtet, sodass sich die ursprüngliche Form inklusive der erfindungsgemäß strukturierten Oberfläche ausbildet.

Die frisch geprägte oder re-hydratisierte Kollagenfolie wird als Kultivierungsmatrix verwendet. Die Oberflächenstruktur der Kultivierungsmatrix ist in der Figur 3 dargestellt. Die Figuren 3A und 3B zeigen REM- Aufnahmen einer oberflächenstrukturierten Gelatinematrix (metacrylierte Gelatine), die mit einem Werkzeug der Topographie 1 geprägt wurde. Die oberflächenstrukturierte Gelatinematrix wurde in vergleichbarer Weise, wie hier exemplarisch für eine Kollagenmatrix beschrieben ist, hergestellt. Figur 3A zeigt eine Gelatinematrix, die mit einem Nickel-Masterwerkzeug abgeformt wurde. Figur 3B zeigt eine Gelatinematrix, die mit einem PDMS-Masterwerkzeug abgeformt wurde.

Sowohl die gegossene oder geprägte Kollagenfolie als auch die geprägte Gelatinematrix lassen sich in getrocknetem Zustand ohne weiteres für mindestens 3 Wochen aufbewahren, so dass nach Zugabe von Wasser oder Kultivierungsmedium beim Quellen der Gele wieder die ursprünglich eingeprägte Oberflächenstrukturierung erhalten wird.

Zur Herstellung neuronaler Zellen werden suspendierte aus adultem Gewebe gewonnene mesenchymale Stammzellen auf die geprägte Gelatinematrix oder Kollagenfolie ausgesät und für vier Wochen kultiviert.

Die kultivierten Zellen entwickeln eine Streckung und parallele Ausrichtung ihrer Morphologie entlang der Mikrostrukturierung der Oberfläche der Kultivierungsmatrix. Rasch verlieren die Zellen ihre ursprüngliche fibroblastenartige Form, und bereits nach wenigen Tagen ist die typische Morphologie neuronaler Zellen erkennbar. Die Zellen entwickeln sehr lange dentritische oder axonische Ausläufer, was deren gute Funktion als Nervenzelle bestimmt.

Das Restkollagen der Kollagen-Matrix wird nach einem Kultivierungszeitraum von vier Wochen mit Hilfe von Kollagenase verdaut, sodass die Zellen schonend und ohne Zerstörung ihrer erlangten Morphologie isoliert werden können. Vitalitätstests zeigen, dass diese Zellen nach den Kollagenase-Verdau keine Einschränkung Ihrer Vitalität aufweisen. In Assays wurden auf Gen- und Proteinebene neuronale Marker in den Zellen nachgewiesen.

## Patentansprüche

1. Verfahren zur Herstellung einer Matrix zur Kultivierung von biologischen Zellen und Differenzierung in neuronale Zellen oder neuronales Gewebe, aus einem Grundkörper (10) aus biokompatiblem oder biologischem Polymer mit einer strukturierten Oberfläche (20), aufweisend
- eine periodische Mikrostruktur (22) aus parallelen mikrodimensionierten Nuten (23) und
- eine periodische Nanostruktur (26) aus parallelen nanodimensionierten Nuten (27), die zu der Mikrostruktur (22) parallel verläuft und jeweils in die Nuten (23) der Mikrostruktur (22) eingebettet ist, enthaltend die Schritte:
- Ausbilden einer strukturierten Oberfläche an einem Werkzeug (30) durch spanabhebende Bearbeitung, wobei die spanabhebende Bearbeitung mit einem Schneidwerkzeug erfolgt, das eine Schneide aus monokristallinem Diamant mit einen Spitzenradius von 10 µm oder weniger aufweist und die periodische Mikrostruktur (22) und die darin eingebettete periodische Nanostruktur (26) als Negativform gebildet werden, und
- Erzeugen der strukturierten Oberfläche (20) an dem Grundkörper (10) aus biokompatiblen oder biologischem Polymer durch:
- Aufgießen des Polymers des Grundkörpers (10) auf das oberflächenstrukturierte Werkzeug (30)
oder
- Prägen des Polymers des Grundkörpers (10) mit dem oberflächenstrukturierten Werkzeug (30) und
- gegebenenfalls Aushärten oder Trocknen des Polymers des Grundkörpers (10) an dem Werkzeug (30) und Abtrennen des ausgehärteten, trockenen Grundkörpers (12) von dem Werkzeug (30),
so dass die Matrix als der Grundkörper (10) mit der strukturierten Oberfläche (20) erhalten wird.

2. Verfahren nach Anspruch 1, wobei das Polymer ausgewählt ist aus Kollagen, Gelatine, Agarose, Derivaten und halbsynthetischen Modifikationen davon.

3. Verfahren nach Anspruch 1 oder 2, wobei das Polymer an der strukturierten Oberfläche eine Steifigkeit ausweist, die der Steifigkeit der dort zu kultivierenden biologischen Zellen entspricht oder nicht größer als diese ist.

4. Verfahren nach einem der vorstehenden Ansprüche, wobei die Mikrostruktur (22) aus Nuten (23) mit einer Tiefe von 0,7 bis 1,5 µm und einer Breite von 1 bis 2 µm, die in einem Mittenabstand von 3 bis 12 µm zueinander liegen, gebildet ist, und wobei die Nanostruktur (26) aus Nuten (27) mit einer Tiefe von 15 bis 35 nm und einer Breite von 450 bis 550 nm, die in einem Mittenabstand von 500 bis 550 nm zueinander liegen, gebildet ist.

5. Verfahren nach einem der vorstehenden Ansprüche, wobei das Erzeugen der strukturierten Oberfläche (20) an dem Grundkörper (10) durch Prägen des Polymers des Grundkörpers (10) mit einem als Prägewalze ausgebildeten oberflächenstrukturierten Werkzeug (30) erfolgt.

## Claims

1. A method of preparing a matrix for cultivating biological cells and differentiating into neuronal cells or neuronal tissue, from a base body (10) of biocompatible or biological polymer having a structured surface (20), comprising
- a periodic microstructure (22) of parallel microdimensioned grooves (23), and
- a periodic nanostructure (26) of parallel nanodimensioned grooves (27) parallel to the microstructure (22) and embedded into the grooves (23) of the microstructure (22), respectively, including the steps of:
- forming a structured surface on a tool (30) by machining, wherein the machining is performed with a machining tool having a cutting edge of monocrystalline diamond with a tip radius of 10 pm or less and the periodic microstructure (22) and the periodic nanostructure (26) embedded therein are formed as a negative shape, and
- producing the structured surface (20) on the base body (10) of biocompatible or biological polymer by:
- casting the polymer of the basic body (10) onto the surface-structured tool (30) or
- embossing of the polymer of the base body (10) with the surface-structured tool (30) and
- optionally curing or drying the polymer of the base body (10) on the tool (30) and separating the cured, dry base body (12) from the tool (30),
such that the matrix is obtained as the base body (10) having the structured surface (20).

2. The method according to claim 1, wherein the polymer is selected from collagen, gelatin, agarose, derivatives and semi-synthetic modifications thereof.

3. The method according to claim 1 or 2, wherein the polymer has a rigidity on the structured surface equal to or no greater than the rigidity of the biological cells to be cultured there.

4. The method according to any one of the preceding claims, wherein the microstructure (22) is formed from grooves (23) having a depth of 0.7 to 1.5 pm and a width of 1 to 2 pm, that have a centre distance of 3 to 12 pm from one another, and wherein the nanostructure (26) is formed from grooves (27) having a depth of 15 to 35 nm and a width of 450 to 550 nm, that have a centre distance of 500 to 550 nm to each other.

5. The method according to any one of the preceding claims, wherein the structured surface (20) is produced on the base body (10) by embossing the polymer of the base body (10) with a surface-structured tool (30) designed as an embossing roller.

## Revendications

1. Un procédé de préparation d'une matrice pour la cultivation de cellules biologiques et leur différenciation en cellules neuronales ou en tissu neuronal, à partir d'un corps de base (10) en polymère biocompatible ou biologique ayant une surface structurée (20), comprenant
- une microstructure périodique (22) de rainures parallèles microdimensionnées (23), et
- une nanostructure périodique (26) de rainures nanodimensionnées parallèles (27) parallèles à la microstructure (22) et intégrées dans les rainures (23) de la microstructure (22), respectivement, comprenant les étapes consistant à :
- former une surface structurée sur un outil (30) par usinage, dans lequel l'usinage est réalisé avec un outil d'usinage ayant un bord de coupe en diamant monocristallin avec un rayon de pointe de 10 pm ou moins et la microstructure périodique (22) et la nanostructure périodique (26) intégrée dans celle-ci sont formées comme une forme négative, et
- la production de la surface structurée (20) sur le corps de base (10) en polymère biocompatible ou biologique par :
- coulant le polymère du corps de base (10) sur l'outil à surface structurée (30) ou
- gaufrage du polymère du corps de base (10) avec l'outil à surface structurée (30) et
- optionnellement, le durcissement ou le séchage du polymère du corps de base (10) sur l'outil (30) et la séparation du corps de base durci et sec (12) de l'outil (30),
de sorte que la matrice est obtenue en tant que corps de base (10) ayant la surface structurée (20).

2. Le procédé selon la revendication 1, dans lequel le polymère est choisi parmi le collagène, la gélatine, l'agarose, leurs dérivés et leurs modifications semi-synthétiques.

3. Le procédé selon la revendication 1 ou 2, dans lequel le polymère a une rigidité sur la surface structurée égale ou non supérieure à la rigidité des cellules biologiques à y cultiver.

4. Le procédé selon l'une quelconque des revendications précédentes, dans lequel la microstructure (22) est formée de rainures (23) ayant une profondeur de 0,7 à 1,5 pm et une largeur de 1 à 2 pm, qui ont une distance centrale de 3 à 12 pm les unes des autres, et dans lequel la nanostructure (26) est formée de rainures (27) ayant une profondeur de 15 à 35 nm et une largeur de 450 à 550 nm, qui ont une distance centrale de 500 à 550 nm les unes des autres.

5. Le procédé selon l'une quelconque des revendications précédentes, dans lequel la surface structurée (20) est produite sur le corps de base (10) par gaufrage du polymère du corps de base (10) avec un outil à surface structurée (30) conçu comme un rouleau de gaufrage.
